# EUROPEAN PATENT SPECIFICATION

(11) **EP 3 321 670 B1**
(45) Date of publication and mention of the grant of the patent: **17.06.2020**
(21) Application number: 16820885.8
(22) Date of filing: 19.04.2016
(51) Int. Cl.: G01N 27/327, C12Q 1/26, C12Q 1/28, C12Q 1/00, C12M 1/34, C12M 1/40, C12N 9/08, G01N 33/02

(54) **SYSTEM AND METHOD FOR MEASURING SULPHITE IN FOOD SAMPLES USING AN AMPEROMETRIC BIOSENSOR AND THE USE OF SAID BIOSENSOR**
SYSTEM UND VERFAHREN ZUR MESSUNG VON SULFIT IN LEBENSMITTELPROBEN UNTER VERWENDUNG EINES AMPEROMETRISCHEN BIOSENSORS UND VERWENDUNG DIESES BIOSENSORS
SYSTÈME ET MÉTHODE POUR MESURER LES SULFITES DANS DES ÉCHANTILLONS D'ALIMENTS AU MOYEN D'UN BIOCAPTEUR AMPÉROMÉTRIQUE ET UTILISATION DUDIT BIOCAPTEUR

(30) Priority: 09.07.2015 ES 201530995
(43) Date of publication of application: 16.05.2018
(73) Proprietor: Biolan Microbiosensores, S.L., 48170 Zamudio (Bizkaia) (ES)
(72) Inventor: GONZALEZ RIOJA, Roberto, 48170 Zamudio (Bizkaia) (ES); MAZA DEL RIO, Sonia, 48170 Zamudio (Bizkaia) (ES); SALLERES ALONSO, Sandra, 48170 Zamudio (Bizkaia) (ES); JAUREGUIBEITIA CAYROLS, Arrate, 48170 Zamudio (Bizkaia) (ES); GONZALEZ URQUIDI, Irune, 48170 Zamudio (Bizkaia) (ES); ARANTZAMENDI EGIGUREN, Alai, 48170 Zamudio (Bizkaia) (ES)
(74) Representative: Igartua, Ismael
(86) International application number: PCT/ES2016/070279
(87) International publication number: WO 2017/005947

(56) References cited:
- WO-A2-2010/010211
- ES-A1- 2 524 991
- US-A- 4 677 059
- US-A1- 2006 278 537
- US-B1- 6 897 035
- US-B2- 8 758 591
- ROBERTO SPRICIGO ET AL: "Electrocatalytic sulfite biosensor with human sulfite oxidase co-immobilized with cytochrome c in a polyelectrolyte-containing multilayer", ANALYTICAL AND BIOANALYTICAL CHEMISTRY, SPRINGER, BERLIN, DE, vol. 393, no. 1, 21 October 2008 (2008-10-21), pages 225-233, XP019652971, ISSN: 1618-2650
- NICULESCU M ET AL: "Redox hydrogel-based amperometric bienzyme electrodes for fish freshness monitoring", ANALYTICAL CHEMISTRY, AMERICAN CHEMICAL SOCIETY, US, vol. 72, no. 7, 1 April 2000 (2000-04-01), pages 1591-1597, XP002954039, ISSN: 0003-2700, DOI: 10.1021/AC990848+
- DELVAUX M ET AL: "Bienzyme HRP-GOx-modified gold nanoelectrodes for the sensitive amperometric detection of glucose at low overpotentials", BIOSENSORS AND BIOELECTRONICS, ELSEVIER SCIENCE LTD. UK, AMSTERDAM, NL, vol. 20, no. 8, 15 February 2005 (2005-02-15), pages 1587-1594, XP027619506, ISSN: 0956-5663 [retrieved on 2005-02-15]
- GROOM C A ET AL: "Development of a flow injection analysis mediated biosensor for sulfite", JOURNAL OF BIOTECHNOLOGY, ELSEVIER, AMSTERDAM, NL, vol. 27, no. 2, 1 January 1993 (1993-01-01), pages 117-127, XP023788210, ISSN: 0168-1656, DOI: 10.1016/0168-1656(93)90102-S [retrieved on 1993-01-01]
- HENDRY S P ET AL: "Amperometric biosensors", JOURNAL OF BIOTECHNOLOGY, ELSEVIER, AMSTERDAM, NL, vol. 15, no. 3, 1 August 1990 (1990-08-01) , pages 229-238, XP023943958, ISSN: 0168-1656, DOI: 10.1016/0168-1656(90)90029-B [retrieved on 1990-08-01]
- MULCHANDANI A ET AL.: 'Determination of sulfite in food products by an enzyme electrode.' JOURNAL OF BIOTECHNOLOGY vol. 18, no. 1-2, AMSTERDAM, NL, ISSN 0168-1656 pages 93 - 102, XP023944284
- MATSUMOTO K ET AL.: 'Determination of sulfite in white wine by amperometric flow injection analysis with an immobilized sulfite oxidase reactor.' AGRICULTURAL AND BIOLOGICAL CHEMISTRY vol. 53, no. 9, 1989, pages 2347 - 2354, XP055507497
- DINCKAYA ET AL.: 'Sulfite determination using sulfite oxidase biosensor based glassy carbon electrode coated with thin mercury film.' FOOD CHEMISTRY vol. 101, no. 4, 01 January 2007, NL, ISSN 0308-8146 pages 1540 - 1544, XP005710465

## Description

### STATE OF THE ART

Sulphites are food additives with antioxidant and antimicrobial properties that are used in the food industry to improve quality, appearance and to extend the shelf life of foods and drinks. In crustaceans, sulphites are applied after the harvest and during the entire production process to prevent melanosis. The addition of sulphites inhibits the formation of black stains caused by the enzymatic oxidation of the monophenols to melanin. Melanosis does not affect the taste of crustaceans and is not harmful for consumers. Nevertheless, the aforementioned stains can drastically affect the acceptability of the products by the consumer and significantly decrease their market value.

The community legislation on food additives is based on the principle that only the additives that are explicitly authorized can be used. The majority of the food additives can only be used in limited quantities in specific food products and they are listed in Regulation No. 1129/2011, which modifies the CE no. 1333/2008. This Regulation lists the food additives with maximum levels allowed different from *quantum satis.* For fresh and frozen crustaceans and cephalopods the maximum concentration of sulphite (E220-E228) allowed is 150 mg/kg.

On the other hand, the Commission of the Codex Alimentarius names sulphites as priority allergens and indicates that the control of the use of sulphite is important to protect the consumers who are sensitive to sulphite.

In food and drinks, sulphite is bonded reversibly in the form of adducts with carbonyl compounds and hydroxy sulphonates. These adducts are stable to low and intermediate pH and disassociate completely free sulphite above pH 9. The free and reversible forms of sulphite bond are referred to as total sulphite. Both in free and total form, both are of interest for the food industry.

A critical control point in the processing of crustaceans is established to guarantee that the final product does not contain an excessive concentration of sulphite. The critical limits must be established in the entire process to ensure that the final product complies with the regulatory limits and the periodic supervision of the effectiveness of the treatment and the controls must be required. Therefore, the monitoring of sulphite must be an integral part of a program of the Firm of Hazard Analysis and Critical Control Points (HACCP).

The HACCP needs to be rapid and optimal to be applied daily, but the current methods for the determination of sulphite are complicated, are time-consuming and are subjective.

Said methods such as test strips, enzymatic methods or methods based on titration have great limitations, false positives, and little reproducibility and accuracy.

The optimized Monier-Williams method (AOAC 962.16) has been fully utilized to determine the total concentration of sulphites in food products. Nevertheless, this method involves much time in analysis, labour, it requires technical personnel and shows false positives.

Amperometric biosensors having a three-electrode (working/ counter/ reference) configuration for the determination of sulphite are known from the prior art. The document" Electrocatalytic sulphite biosensor with human sulphite oxidase co-immobilized with cytochrome c in a polyelectrolyte-containing multilayer" by R. Spricigo et al discloses an amperometric biosensor for sulphite where the sulphite oxidase is co-immobilized with cytochrome-c and polyelectrolyte to build a multi-layered structure. ES 2524991 A1 discloses an amperometric biosensor for sulphite where the working electrode incorporates 3% tetrathiafulvalene (TTF), on a polyester plastic sheet, modifying the active area of the work with the enzyme sulphite oxidase (SOx) on its surface by crosslinking with glutaraldehyde (GA) and bovine serum albumin (BSA). The document "Development of a flow injection analysis mediated biosensor for sulphite" by Groom et al discloses an biosensor for sulphite where the sulphite oxidase and a mediator are deposited onto a sensing area of a vitreous carbon electrode which was then covered by a dialysis membrane.

Amperometric biosensors not related with the measurement of sulphite, with one enzyme which is covered by a dialysis membrane are known from WO 2010/010211 A1.

Amperometric biosensors based on bienzymatic systems including a peroxidase but not concerned with the measurement of sulphite are also known from US 2006/0278537 A1, the document" Redox Hydrogel-Based Amperometric Bienzyme Electrodes for Fish Freshness Monitoring" by Niculescu et al and the document "Design of Biosensors with Extended Linear Response and Binary-Type Sigmoid Output Using Multiple Enzymes" by Delvaux et al. Therefore, one of the great challenges of the industry of crustaceans is to achieve a system that is highly sensitive, fast and portable for the determination of sulphite in crustaceans in order to prevent contaminated products from reaching the market. Enzymatic biosensors are suitable solutions that will be applied for the determination of sulphites in food, more specifically in crustaceans, as simple, precise and fast cost tools.

### OBJECT OF THE INVENTION

One object of the invention is an amperometric biosensor according to claim 1.

The method for the determination of sulphite in food samples using the aforementioned biosensor according to claim 7 is also another object of the invention.

The use of the amperometric biosensor to measure the values of sulphite in food samples is also another object of the invention.

In particular, the food samples are crustaceans.

Today, a commercial biosensor of sulphite applied to the agro-food industry does not exist, and no commercial enzyme is available.

The system which is the object of the invention offers results which are as reliable, or more so, than the method of (Monier-Williams) used today, but with advantageous characteristics/particularities with respect to it which are reflected in summarized form in the following table:

| | Optimized Monier-Williams method (AOAC 962.16) | Object of the invention |
|---|---|---|
| Procedure | Acid distillation followed by titration | Enzymatic oxidation of the sulphite, amperometric detection |
| Time of analysis | 1 hour | 3 Minutes |
| Simplicity | Low | Medium |
| Interpretation | Comparison with the standards at the start of the titration | Result on screen |
| Limitation | Limited quantification | Daily calibration |

### DESCRIPTION OF THE DRAWINGS

To better understand the object of the invention, a preferential form of embodiment is represented in the attached figures, subject to accessory changes that do not essentially alter it. In this case:
Figure 1 represents the reaction scheme for the determination of sulphite.
Figure 2 represents the calibration curve to see the range of response of the biosensor to sulphite.

### DETAILED DESCRIPTION OF THE INVENTION

This invention represents a system for measuring sulphite in food samples by biosensor; a method for the determination of sulphite in food samples using the aforementioned biosensor; and the use of the aforementioned biosensor to measure the values of sulphite in food samples.

In particular, the food samples are crustaceans.

The biosensor used is an amperometric biosensor that comprises a bi-enzymatic system, which is composed by two enzymes: humane sulphite oxidase (EC 1.3.8.1) and peroxidase.

The aforementioned amperometric biosensor is composed of a substrate, a working electrode, a counter electrode and a reference electrode. Preferably, the biosensor is composed of a gold working electrode, a stainless-steel counter electrode and of a silver/silver chloride(Ag/AgCl). reference electrode.

The enzymes are deposited on the surface of the working electrode with simple physical retention. Before depositing them, on the surface of the working electrode the chemical mediator is deposited which can comprise mediators such as Methylene Blue, Meldola's Blue, Tetrathiafulvalene, Ferricyanide and Ferrocene, dissolved in organic solvents such as isopropyl alcohol, propanol, methanol and chloroform. The chemical mediator is preferably Ferrocene dissolved in isopropyl alcohol.

The method which is the object of the invention comprises the phases of
a) application of a sample to the amperometric biosensor;
b) change of electric current on entering into contact with the analyte to be detected; and
c) measuring amperometrically said electric current in solutions with continuous stirring, using a constant potential.

Before depositing the enzymes on the working electrode, these are dissolved in a carbonate/bicarbonate buffer and/or CAPS and/or CAPSO 0.1 M in a pHs range of 8.0-11.1, the optimal pH being 10. For said preparation, sulphite oxidase:peroxidase ratios of 1:1, 1 :2, 1:3 and 1:4 are worked with in terms of enzymatic units, the optimal ration being 1:3.

Said bi-enzymatic combination, in turn, comprises a drying solution composed of trehalose and/or monosodium glutamate in a range of 10-40%, the optimal value being 30%.

The enzymes deposited on the working electrode are covered with a permeable or osmotic membrane such as those used in dialysis, previously hydrated, with a nominal molecular weight limit in the range of 5-50 kilodaltons (kDa), the optimal range being 6-10 kDa.

Subsequently, for its use, the biosensor is hydrated in the measuring solution that corresponds to the carbonate/bicarbonate buffer 0.1 M in a pHs range of 8.0-11.1, the optimal pH being 10.

The biocatalytic scheme for the determination of sulphite in crustaceans would be as follows:
At pH 10 all the sulphur dioxide present in the sample (both free and combined) is in the form of S03⁻ (sulphite form).

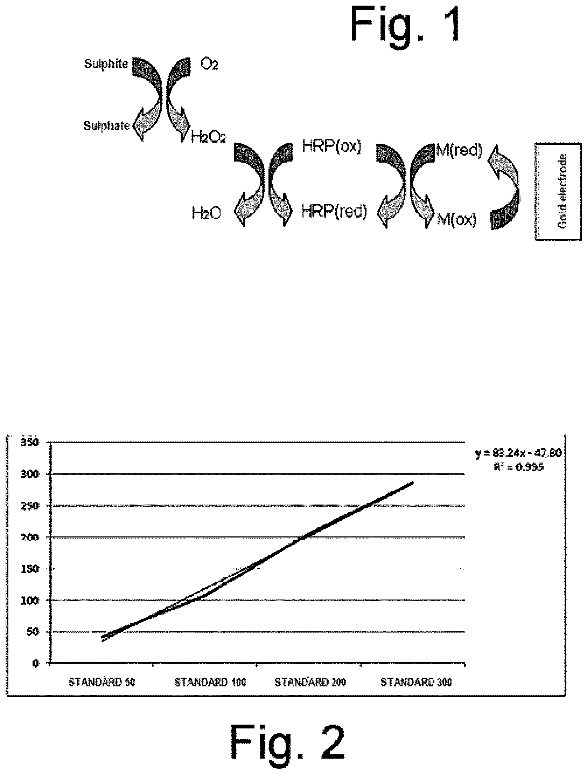

The enzyme sulphite oxidase mediates in the oxidation of the sulphite to sulphate generating hydrogen peroxide in presence of oxygen. The subsequent catalytic reduction of the peroxide generated by the peroxidase (HRP), is the indirect measure of the oxidized sulphite. The ferrocene is used as a chemical mediator for this last enzymatic reaction; therefore, the amperometric signal that is used for the reading of this scheme of reactions corresponds to the reduction of said mediator, as indicated in figure 1, which represents a reaction scheme for the determination of sulphite.

The level of sulphite in a sample is determined applying the sample to the biosensor and measuring the signal of current to a potential that is found in the range of 0 to 50 mV. Preferably, the voltage of the electric current used for the determination of sulphite is approximately 0 mV.

The pH for the determination of sulphite is in the range of 8.0 to 11.1. Preferably, the pH for the determination of sulphite is approximately 10.0.

Said biosensor together with a measuring cuvette and a stirrer is what we call an electrochemical cell which consists of the following elements:
- Working electrode;
- Counter electrode;
- Reference electrode;
- Measuring cuvette; and
- Stirrer

The biosensor has a range of response of 50-300 ppm of sulphite; and in addition, the linear response is total in said range, as shown in figure 2, which represents the range of response of the biosensor (calibration curve of 50 to 300 mg/kg). Thus, the ranged marked by Regulation No. 1129/2011 is covered, which lists the food additives with maximum levels allowed for fresh and frozen crustaceans and cephalopods; said value being 150 ppm.

A calibration line is constructed of 5 points (not including the white), and of 3 replicas for each concentration. The linearity is evaluated, checking the regression coefficient r2 and the Response/Concentration Factor.

Once the linearity is verified in the range of 50 to 300 mg/kg, it is studied whether routine calibrations made by the biosensor (2 points) are stable enough to ensure accuracy and precision during the lifetime of the biosensor. For this purpose, 23 calibrations were made with different biosensors, so that the stability of the slope (m), of the coefficient of linear regression (r²) was checked. The criteria of acceptance proposed are as follows:
- The % RSD of the slope will not exceed 15 %
- No straight line will have a r²< 0,995 (R < 0.99)

The results obtained are shown on the following table:

| BIOSENSOR | r² | SLOPE (m) | CUT-OFF POINT (b) |
|---|---|---|---|
| Biosensor 1 | 0.9986 | -268451.72 | - 9.19 |
| Biosensor 1 | 0.9998 | -216795.02 | - 2.08 |
| Biosensor 1 | 0.9997 | -212064.04 | -3.3 |
| Biosensor 1 | 0.9997 | -213370.52 | -3.38 |
| Biosensor 1 | 0.9999 | -216305.44 | -1.96 |
| Biosensor 2 | 0.9999 | -252625.28 | -1.21 |
| Biosensor 2 | 0.9997 | -238793.58 | -3.21 |
| Biosensor 2 | 0.9997 | -260319.96 | -3.89 |
| Biosensor 2 | 0.9999 | -160476.95 | -0.16 |
| Biosensor 2 | 0.9998 | -162145.8 | 1.92 |
| Biosensor 3 | 0.9994 | -158693.02 | -3.36 |
| Biosensor 3 | 0.9998 | -199188.24 | -2.5 |
| Biosensor 3 | 0.9999 | -200175.32 | -1.59 |
| Biosensor 3 | 0.9987 | -207896.84 | -6.96 |
| Biosensor 3 | 0.9994 | -175763.08 | 3.96 |
| Biosensor 4 | 0.9999 | -165459.56 | 0.9 |
| Biosensor 4 | 0.9999 | -241312.66 | -0.55 |
| Biosensor 4 | 0.9999 | -224968.14 | 1.14 |
| Biosensor 4 | 0.9999 | -225537.96 | -0.47 |
| Biosensor 5 | 0.9998 | -254172.28 | -2.75 |
| Biosensor 5 | 0.9998 | -240394.24 | -3.07 |
| Biosensor 5 | 0.9992 | -226618.6 | -5.68 |
| Biosensor 5 | 0.9989 | -217246.88 | -6.48 |
| AVERAGE | 0.9996 | -214729.35 | -2.34 |
| SD | 0.0004 | 32738.83 | 3 |
| RSD(%) | 0.04 | 15 | - |
| MAXIMUM | 0.9999 | -158693.02 | 3.96 |
| MINIMUM | 0.9986 | -268451.72 | -9.19 |

The method is considered precise in the entire range of work, from 50 to 300 mg/kg and in the matrices that are the test object (raw and cooked prawn), according to the criteria of RSO HORWITZ.

During the lifetime of the biosensor, the stability of the straight lines of calibration is considered suitable.

The accuracy is the degree of concordance between the result of the test and an accepted reference value. Said accuracy is obtained by the study of recoveries, in the entire range of work, from 50 to 300 mg/kg, so that at each level of concentration, the two matrices of study are analysed (raw and cooked prawn). The method is considered of good accuracy if the average recovery at all the levels of concentration, meets the criteria established by AOAC. The following table shows the results:

| PLANINNG AND RESULTS | | | ACCURACY | | |
|---|---|---|---|---|---|
| | | | RECOVERY | | |
| REFERENCE VALUE (mg/kg) | MATRIX | No. Replicas | % AVG. REC. | CRITERIA | Acceptance |
| 50 | RAW PRAWN | 4 | 84 | 80-110 | YES |
| | COOKED PRAWN | 4 | | | |
| 100 | RAW PRAWN | 4 | 96 | 90-107 | YES |
| | COOKED PRAWN | 4 | | | |
| 200 | RAW PRAWN | 4 | 92 | 90-107 | YES |
| | COOKED PRAWN | 4 | | | |
| 300 | RAW PRAWN | 4 | 96 | 90-107 | YES |
| | COOKED PRAWN | 4 | | | |

The method is considered accurate in the entire range of concentration, from 50 to 300 mg/kg, in the matrices which are the test object (raw and cooked prawn), according to the acceptable intervals of recovery established by AOAC.

On the other hand, the degree of concordance is determined between independent test results obtained under predetermined conditions. Said precision is usually expressed as imprecision by the calculation of relative standard deviations and/or the Horrat ratio. To obtain the maximum imprecision at all the levels of concentration, the maximum variability of matrices (species, fresh and processed products, ...), measured throughout the life of the biosensor, etc. has been considered.

The following table shows the results:

| Sample | Biosensor | Modified Monier-Williams | Interval of uncertainty Modified Monier-Wlliams | Acceptance |
|---|---|---|---|---|
| 1 | 180.9 | 214 | 166-262 | Yes |
| 2 | 195.5 | 224 | 176-272 | Yes |
| 3 | 216 | 236 | 184-288 | Yes |
| 4 | 104.76 | 115 | 90-140 | Yes |
| 5 | 112.06 | 128 | 100-156 | Yes |
| 6 | 137.06 | 127 | 99-155 | Yes |
| 7 | 131.5 | 117 | 92-142 | Yes |
| 8 | 183.82 | 194 | 150-238 | Yes |
| 9 | 81 | 96 | 74-118 | Yes |
| 10 | 68 | 81 | 62-100 | Yes |
| 11 | 156 | 165 | 130-200 | Yes |
| 12 | 155 | 190 | 146-234 | Yes |
| 13 | 97 | 121 | 96-146 | Yes |
| 14 | 121 | 143 | 112-171 | Yes |
| 15 | 120 | 131 | 103-159 | Yes |
| 16 | 226 | 222 | 174-270 | Yes |
| 17 | 181 | 241 | 189-293 | Yes |
| 18 | 96 | 122 | 94-150 | Yes |
| 19 | 155 | 176 | 137-215 | Yes |
| 20 | 117 | 163 | 128-198 | Yes |
| 21 | 127 | 162 | 127-197 | Yes |
| 22 | 165.96 | 202 | 158-246 | Yes |
| 23 | 124.16 | 154 | 119-189 | Yes |
| 24 | 117.16 | 130 | 102-158 | Yes |
| 25 | 107 | 135 | 107-163 | Yes |
| 26 | 60.28 | 55 | 42-68 | Yes |
| 27 | 75.28 | 67 | 51-83 | Yes |
| 28 | 75.18 | 92 | 73-111 | Yes |
| 29 | 294.8 | 337 | 265-409 | Yes |
| 30 | 86.12 | 84 | 65-103 | Yes |
| 31 | 58.16 | 63 | 50-76 | Yes |
| 32 | 61.02 | 75 | 59-91 | Yes |
| 33 | 88.02 | 102 | 80-124 | Yes |
| 34 | 22.34 | 21 | 16-26 | Yes |
| 35 | 41.58 | 46 | 35-57 | Yes |
| 36 | 76.1 | 84 | 65-103 | Yes |
| 37 | 132.2 | 166 | 131-201 | Yes |
| 38 | 172.54 | 206 | 162-250 | Yes |
| 39 | 70.6 | 90 | 71-109 | Yes |
| 40 | 72.52 | 84 | 65-103 | Yes |
| 41 | 65.26 | 75 | 59-91 | Yes |
| 42 | 64.6 | 78 | 62-94 | Yes |
| 43 | 79.84 | 88 | 69-107 | Yes |
| 44 | 70 | 64 | 51-77 | Yes |

The method is considered precise in the whole range of work, from 50 to 300 mg/kg and in the matrices which are the test object (raw and cooked prawn), according to the criteria of RSD HORWITZ.

To ensure that the results obtained are suitable, a comparative study was performed with the modified Monier Williams method for determination of sulphites in samples in which other volatile sulphur compounds are present. It is based on the transformation of the sulphites in SO₂. The SO₂ is oxidized to H₂SO₄ which finally is titrated with a standardized dissolution of NaOH. The modified Monier Williams method is based on the method of the AOAC, Sulphurous Acid (Total) in Food, Optimized Monier-Williams Method. 990.28, pp29, 1995. This method is used frequently in the official control of sulphites in food.

For this purpose, a total of 44 samples of cooked and raw prawn were analysed both by the modified Monier-Williams method and by the method which is the object of the invention. The results obtained are shown in the foregoing table.

The comparative study with the modified Monier-Williams method, shows a reliability value of 95%.

Lastly, the lifetime of the biosensor is determined in relation to its storage and number of measures it can support. For this purpose, a series of biosensors are tested with 0, 15, 30, 60 and 90 days of drying stored at 3-8°C, and hydrated on day 1, 7 and 15 after its drying. The results are shown on the following table:

| | | | |
|---|---|---|---|
| | T0 | | |
| | H1 | H7 | H15 |
| batch calibrat no. of cumulative measures | Biosensor 1 R = 0,9996 | Biosensor 1 R = 0,9999 | Biosensor 1 R = 0,9999 |
| | R = 0,9996 | R = 0,9999 | R = 0,9999 |
| | 110 | 105 | 108 |
| | T15 | | |
| | H1 | H7 | H15 |
| batch calibration no. of cumulative measures | Biosensor 2 | Biosensor 2 | Biosensor 2 |
| | R=0,9999 | R=0,9999 | R=0,9998 |
| | R=0,9999 | R=0,9999 | R=0,9998 |
| | 107 | 110 | 104 |
| | T30 | | |
| | H1 | H7 | H15 |
| batch calibration no. of cumulative measures | Biosensor 3 R = O, 9999 | Biosensor 3 R=0,9999 | Biosensor 3 R= 0,9998 |
| | R = 0, 9999 | R=0,9999 | R= 0,9998 |
| | 104 | 110 | 120 |
| | T60 | | |
| | H1 | H7 | H15 |
| batch calibration no. of cumulative measures | Biosensor 4 | Biosensor 4 | Biosensor 4 |
| | R=0,9993 | R=0,9999 | R=0,9993 |
| | R=0,9993 | R=0,9999 | R=0,9993 |
| | 105 | 105 | 110 |
| | T90 | | |
| | H1 | H7 | H15 |
| batch calibration no. of cumulative measures | Biosensor 2 | Biosensor 2 | |
| | R=0,9999 | R=0,9999 | Biosensor 2 |
| | R=0,9999 | R=0,9999 | R=0,9999 |
| | 115 | 105 | 108 |

| | | | |
|---|---|---|---|
| where: T0= 0 days of dry storage T15= 15 days of dry storage T30= 30 days of dry storage T60= 60 days of dry storage T90= 90 days of dry storage H1= 1 day hydrated H7= 7 days hydrated H15= 15 days hydrated The biosensors are able to support a minimum of 100 measures in any of the aforementioned conditions showing perfect calibration. | | | |

### EXAMPLES OF PREFERENTIAL EMBODIMENT

For a better understanding of this invention, the following examples are shown, described in detail, that must be understood non-limiting of the scope of this invention.

### Example 1. Determination of total sulphite total in raw prawn

Clean and discard the shell, the cephalothorax and other non-edible parts of the exoskeleton and visible digestive tract. Then, homogenise the sample with the aid of a mincer. Weigh 2 gr of the homogenised sample and mix it with 18 mL of extraction solution (buffer 0.1 M CAPS pH 9.5). Stir vigorously 30 seconds and apply high-intensity ultrasound for 20 minutes with 20-second pulses and an amplitude of 60%; allow to reach room temperature before proceeding to analysis.

Add 1 mL of the homogenised sample to the electrochemical cell with 10 mL of buffer 0.1 M CAPS pH 9.5, with constant stirring and start the amperometric measurement in the biosensor at a potential of 0 mV.

### Example 2. Determination of total sulphite in cooked prawn

Clean and discard the shell, the cephalothorax and other non-edible parts of the exoskeleton and visible digestive tract. Then, homogenise the sample with the aid of a mincer. Weigh 5 gr of the homogenised sample and mix it with 40 mL of extraction solution (buffer 0.1 M CAPSO pH 10,0). Stir vigorously 30 seconds and apply low-intensity ultrasound for 30 minutes at 45°C; allow to reach room temperature before proceeding to analysis.

Add 1 mL of the homogenised sample to the electrochemical cell with 10 mL of buffer 0.1 M CAPSO pH 10.0, with constant stirring and start the amperometric measurement in the biosensor at a potential of 0 mV.

### Example 3. Determination of total sulphite in raw prawn

Clean and discard the shell, the cephalothorax and other non-edible parts of the exoskeleton and visible digestive tract. Then, homogenise the sample with the aid of a mincer. Weigh 2 gr of the homogenised sample and mix it with 10 mL of extraction solution (buffer 0.1 M carbonate/bicarbonate pH 10.0). Stir vigorously 30 seconds and apply low-intensity ultrasound for 20 minutes at 40°C; allow to reach room temperature before proceeding to analysis. To eliminate the traces of sample in suspension, proceed to filter/centrifuge the sample before injecting it in the equipment.

Add 1 mL of the homogenised sample to the electrochemical cell with10 mL of buffer 0.1 M carbonate/bicarbonate pH 10.0, with constant stirring and start the amperometric measurement in the biosensor at a potential of 0 mV.

## Claims

1. Amperometric biosensor to measure sulphite in food samples, **characterized in that** it comprises:
a) a bi-enzymatic system that is composed of the enzymes human sulphite oxidase and peroxidase;
b) a working electrode;
c) a counter electrode; and
d) a reference electrode;
wherein the enzymes human sulphite oxidase and peroxidase are deposited on the surface of the working electrode and are covered with a dialysis membrane, and wherein the mixture of the enzymes is in turn, mixed with a chemical mediator.

2. The biosensor according to claim 1, wherein the working electrode is gold.

3. The biosensor according to claim 1, wherein the counter electrode is stainless steel.

4. The biosensor according to claim 1, wherein the reference electrode is a silver chloride (Ag/AgCl) electrode.

5. The biosensor of according to claim 1, wherein the chemical mediator is selected from the list that comprises: methylene blue, tetrathiafulvalene, ferricyanide and ferrocene.

6. The biosensor according to claim 5, wherein the chemical mediator is ferrocene dissolved in isopropyl alcohol.

7. Method for the determination of sulphite in food samples using the amperometric biosensor according to claims 1 to 8, **characterized in that** it comprises the phases of:
a) Contact the sample in measuring solution with the biosensor amperometric;
b) measure amperometrically the electric current that is generated in solutions with continuous stirring, when a constant potential in the range of 0 to 50 mV is applied.

8. The method according to claim 7, wherein the voltage of the electric current used for the determination of sulphite is approximately 0 mV.

9. The method according to claim 7, wherein the pH for the determination of sulphite is in the range of 8.0 to 11.1.

10. The method according to claim 9, in which the pH for the determination of sulphite is approximately 10.0.

11. Use of the biosensor according to claims 1 to 6 to measure the values of sulphite in food samples.

## Patentansprüche

1. Amperometrischer Biosensor zur Messung von Sulfit in Lebensmittelproben, **dadurch gekennzeichnet, dass** er Folgendes umfasst:
a) ein bienzymatisches System, welches aus den Enzymen menschlicher Sulfitoxidase und Peroxidase besteht;
b) eine Arbeitselektrode;
c) eine Gegenelektrode; und
d) eine Bezugselektrode;
wobei die Enzyme menschliche Sulfitoxidase und Peroxidase auf der Oberfläche der Arbeitselektrode abgeschieden werden und mit einer Dialysemembran bedeckt werden, und wobei die Mischung der Enzyme wiederum mit einem chemischen Vermittler gemischt wird.

2. Biosensor nach Anspruch 1, wobei die Arbeitselektrode Gold ist.

3. Biosensor nach Anspruch 1, wobei die Gegenelektrode Edelstahl ist.

4. Biosensor nach Anspruch 1, wobei die Bezugselektrode eine Elektrode aus Silberchlorid (Ag/AgCl) ist.

5. Biosensor nach Anspruch 1, wobei der chemische Vermittler aus der Liste ausgewählt wird, welche Folgendes umfasst: Methylenblau, Tetrathiafulvalen, Ferricyanid und Ferrocen.

6. Biosensor nach Anspruch 5, wobei der chemischen Vermittler in Isopropylalkohol gelöstes Ferrocen ist.

7. Verfahren zur Bestimmung von Sulfit in Lebensmittelproben unter Verwendung des amperometrischen Biosensors nach den Ansprüchen 1 bis 8, **dadurch gekennzeichnet, dass** es die folgenden Phasen umfasst:
a) das Kontaktieren der Probe in der Messlösung mit dem amperometrischen Biosensor;
b) das amperometrische Messen des elektrischen Stroms, welcher in Lösungen mit kontinuierlichem Rühren erzeugt wird, wenn ein konstantes Potential im Bereich von 0 bis 50 mV angelegt wird.

8. Verfahren nach Anspruch 7, wobei die Spannung des elektrischen Stroms, welcher zur Bestimmung von Sulfit verwendet wird, etwa 0 mV ist.

9. Verfahren nach Anspruch 7, wobei der pH-Wert zur Bestimmung von Sulfit im Bereich von 8.0 bis 11.1 liegt.

10. Verfahren nach Anspruch 9, in welchem der pH-Wert zur Bestimmung von Sulfit etwa 10.0 ist.

11. Verwendung des Biosensors nach den Ansprüchen 1 bis 6, um die Sulfitwerte in Lebensmittelproben zu messen.

## Revendications

1. Biocapteur ampérométrique pour mesurer le sulfite dans des échantillons d'aliments, **caractérisé en ce qu'**il comprend:
a) un système bi-enzymatique qui est composé des enzymes sulfite oxydase humaine et peroxydase;
b) une électrode de travail;
c) une contre-électrode; et
d) une électrode de référence;
dans lequel les enzymes sulfite oxydase humaine et peroxydase sont déposées sur la surface de l'électrode de travail et sont couvertes par une membrane de dialyse, et dans lequel le mélange des enzymes est à son tour mélangé avec un médiateur chimique.

2. Biocapteur selon la revendication 1, dans lequel l'électrode de travail est de l'or.

3. Biocapteur selon la revendication 1, dans lequel la contre-électrode est de l'acier inoxydable.

4. Biocapteur selon la revendication 1, dans lequel l'électrode de référence est une électrode en chlorure d'argent (Ag/AgCl).

5. Biocapteur selon la revendication 1, dans lequel le médiateur chimique est choisi parmi la liste qui comprend : bleu de méthylène, tétrathiafulvalène, ferricyanure et ferrocène.

6. Biocapteur selon la revendication 5, dans lequel le médiateur chimique est du ferrocène dissout dans de l'alcool isopropylique.

7. Méthode pour la détermination de sulfite dans des échantillons d'aliments au moyen du biocapteur ampérométrique selon les revendications 1 à 8, **caractérisé en ce qu'**il comprend les étapes de:
a) mettre en contact l'échantillon dans une solution de mesure avec le biocapteur ampérométrique;
b) mesurer de manière ampérométrique le courant électrique qui est généré dans des solutions en brassage continu, lorsqu'un potentiel constant dans l'intervalle de 0 à 50 mV est appliqué.

8. Méthode selon la revendication 7, dans lequel la tension du courant électrique utilisé pour la détermination de sulfite est environ 0 mV.

9. Méthode selon la revendication 7, dans lequel le pH pour la détermination de sulfite se trouve dans l'intervalle de 8.0 à 11.1.

10. Méthode selon la revendication 9, dans lequel le pH pour la détermination de sulfite est environ 10.0.

11. Utilisation du biocapteur selon les revendications 1 à 6 pour mesurer les valeurs de sulfite dans des échantillons d'aliments.
